(19) Europäisches Patentamt
European Patent Office
Office européen des brevets

(11) Veröffentlichungsnummer : **0 008 653**
**B2**

(12) # NEUE EUROPÄISCHE PATENTSCHRIFT

(45) Veröffentlichungstag der neuen Patentschrift : **04.05.88**

(51) Int. Cl.⁴ : **C 07 D235/06**, A 61 K 31/41

(21) Anmeldenummer : **79102580.2**

(22) Anmeldetag : **21.07.79**

(54) **N-Substituierte Benzimidazole, Verfahren zu ihrer Herstellung, und sie enthaltende Arzneimittel.**

(30) Priorität : **28.07.78 DE 2833140**

(43) Veröffentlichungstag der Anmeldung : **19.03.80 Patentblatt 80/06**

(45) Bekanntmachung des Hinweises auf die Patenterteilung : **16.06.82 Patentblatt 82/24**

(45) Bekanntmachung des Hinweises auf die Entscheidung über den Einspruch : **04.05.88 Patentblatt 88/18**

(84) Benannte Vertragsstaaten :
**AT BE CH DE FR GB IT LU NL SE**

(56) Entgegenhaltungen :
**DE-A- 1 643 224**
**DE-A- 1 934 037**
**DE-A- 2 135 678**
**DE-A- 2 238 504**
**DE-A- 2 644 833**
**FR-A- 2 343 431**
**US-A- 3 274 194**
**US-A- 3 718 648**
**US-A- 4 154 829**
**US-A- 4 378 361**
**Journal of Medicinal Chemistry, 1970, Vol. 13, No. 4, pp. 674-680**
**Pharm. Ther. B. Vol. 2 pp. 423-462, 1976**
**Arzneim. Forschung/Drug Res. 27 (I) Nr. 1a (1977) pp. 4-34**
**Die Akte enthält technische Angaben, die nach dem Eingang der Anmeldung eingereicht wurden und die nicht in dieser Patentschrift enthalten sind.**

(73) Patentinhaber : **C.H. BOEHRINGER SOHN**
**Postfach 200**
**D-6507 Ingelheim am Rhein (DE)**

(72) Erfinder : **Schromm, Kurt, Dr.**
**In der Dörrwies 35**
**D-6507 Ingelheim (DE)**
Erfinder : **Mentrup, Anton, Dr.**
**Steinernstrasse 25**
**D-6508 Mainz-Kastel (DE)**
Erfinder : **Renth, Ernst-Otto, Dr.**
**Frankenstrasse 11**
**D-6507 Ingelheim (DE)**
Erfinder : **Fügner, Armin, Dr.**
**Im Hippel 31**
**D-6535 Gau-Algesheim (DE)**
Erfinder : **Streller, Ilse, Dr.**
**Stromberger Strasse 6a**
**D-6531 Dörrebach (DE)**

**EP 0 008 653 B2**

Jouve, 18, rue St-Denis, 75001 Paris, France

**Beschreibung**

Die Erfindung betrifft N-substituierte Benzimidazole, ihre Herstellung nach an sich bekannten Verfahren und ihre Verwendung in Form von Arzneimitteln.

Die neuen Verbindungen entsprechen der allgemeinen Formel

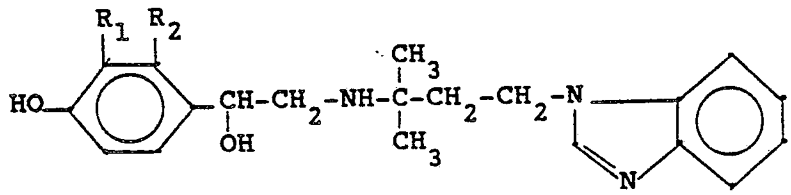

(I)

in der

$R_1$ $CH_2OH$, $NHSO_2CH_3$, $NHCOCH_3$, $NHCONH_2$ und CN bedeutet und $R_2$ für H steht oder

$R_1$ H bedeutet und $R_2$ für F oder Cl steht oder

$R_1$ und $R_2$ gemeinsam für $—O—CH_2—CO—NH—$ in 2,3-Position stehen, in Form der Racemate oder Enantiomeren und der jeweiligen Säureadditionssalze.

Hervorzuheben sind die Verbindungen, in denen $R_1/R_2$ die Substituentenkombinationen $CH_2OH/H$, $NHCONH_2/H$, H/F oder H/Cl bedeuten, insbesondere diejenige Verbindung, in der $R_1$ Wasserstoff, $R_2$ Fluor bedeutet, jeweils in Form des Racemats, der Enantiomeren und der Säureadditionssalze.

Verbindungen der Formel I sind bisher nicht beschrieben worden.

In der DE-A 1 643 224 ist zwar eine allgemeine Formel aufgestellt worden, in der die an den Stickstoff der Seitenkette gebundene Alkylgruppe $R_3$ durch heterocyclische Ringe mit mindestens einem Heteroatom substituiert sein kann. Die Art der heterocyclischen Ringe ist jedoch nicht näher definiert, und als Beispiel ist lediglich die Morpholinogruppe aufgeführt. Ein Hinweis auf die Benzimidazolgruppe gemäß der Erfindung ist in der Anmeldung nicht zu finden. Von den Verbindungen dieser Anmeldung ist ein N-tert.-Butylderivat (SALBUTAMOL) als Broncholytikum in den Handel gekommen. Die erfindungsgemäßen Verbindungen haben sich gegenüber diesem Handelsprodukt im Test als überlegen erwiesen.

Die DE-A 2 238 504 betrifft verwandte Verbindungen mit β-blockierender Wirkung ; sie weisen die für viele β-Blocker typische 1-Phenoxy-2-ol-3-amino-Struktur auf. Demgegenüber zeigen die erfindungsgemäßen Verbindungen β-mimetische Wirkung. Eine Übertragung der Struktur/Wirkungsbeziehungen aus der Entgegenhaltung auf die erfindungsgemäßen Verbindungen ist daher nicht möglich.

In der DE-A 2 135 678 sind Verbindungen erwähnt, die anstelle des Benzimidazolrestes der erfindungsgemäßen Verbindungen einen substituierten Phenylring enthalten. Es gibt indessen keinen Anhaltspunkt dafür, daß ein solcher Phenylrest einem Benzimidazolrest äquivalent wäre.

In der Veröffentlichung von Klinger, Arzneim.-Forsch., Drug Res. 27, I (1977), S. 4ff. und 22ff., sind Xanthin-, insbesondere Theophyllinderivate beschrieben, die eine Phenylethanolamingruppe enthalten. Diese Verbindungen sind jedoch, wie durch Vergleichsversuche gezeigt werden konnte, durch ein weniger günstiges Verhältnis von intravenöser und oraler Wirkung gekennzeichnet. Außerdem ist für die Verbindungen der Veröffentlichung eine tokolytische Wirkung nicht angegeben. Eine für die Praxis interessante tokolytische Wirkung kann auch nicht ohne weiteres angenommen werden, da das Verhältnis Broncholyse/Tokolyse bei β-Mimetika stark differiert.

Die in der FR-A-2 343 731 (= US-A-4 154 829) beschriebenen strukturähnlichen Verbindungen erweisen sich ebenfalls in der pharmakologischen Untersuchung als den erfindungsgemäßen Verbindungen unterlegen. Auch in der FR-A findet sich kein Hinweis auf eine tokolytische Wirkung der dort beschriebenen Verbindungen.

Die erfindungsgemäßen Verbindungen können in an sich bekannter Weise hergestellt werden, indem man

a) ein Aminoketon der allgemeinen Formel

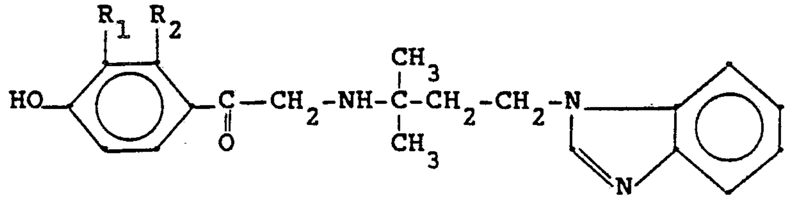

(II)

in der $R_1$ und $R_2$ die oben angegebene Bedeutung haben und in der das zentrale Stickstoffatom und/oder die phenolische OH-Gruppe durch hydrogenolytisch abspaltbare Schutzgruppen geschützt sein können,

in einem geeigneten Lösungsmittel mit Wasserstoff und Hydrierungskatalysatoren oder mit reduzierend wirkenden Hydriden reduziert und gegebenenfalls nach der Reduktion noch vorhandene Schutzgruppen hydrogenolytisch entfernt oder indem man

b) das Amin der Formel

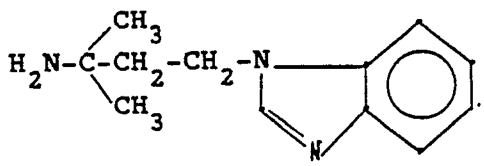

(V)

unter den Bedingungen der reduktiven Aminierung mit einem Phenylglyoxal der allgemeinen Formel

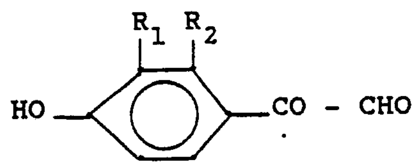

(VI)

in der $R_1$ und $R_2$ die obige Bedeutung haben und in der die phenolische OH-Gruppe durch eine hydrogenolytisch abspaltbare Schutzgruppe geschützt sein kann, gegebenenfalls in Form eines Halbacetals umsetzt und gegebenenfalls die Schutzgruppe hydrogenolytisch abspaltet oder daß man

c) aus einer Verbindung der allgemeinen Formel

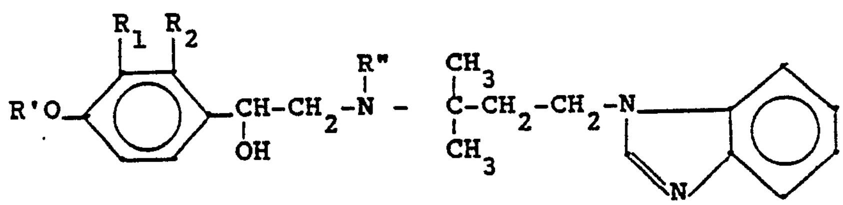

(IX)

in der $R_1$ und $R_2$ die obige Bedeutung haben, R' und R" für H oder eine hydrogenolytisch abspaltbare Schutzgruppe stehen, wobei mindestens einer der Reste R' und R" eine Schutzgruppe bedeutet, die Schutzgruppe(n) entfernt

und daß man die nach a) bis c) erhaltenen Verbindungen gewünschtenfalls in die Enantiomeren auftrennt und daß man gewünschtenfalls erhaltene Basen in Säureadditionssalze, erhaltene Säureadditionssalze in die freien Basen oder in Salze mit anderen Säuren überführt.

Bei Verfahren a) verwendet man Lösungsmittel, die unter den Reaktionsbedingungen ausreichend inert sind, z. B. Alkohole wie Methanol, Äthanol, und übliche Hydrierungskatalysatoren, beispielsweise Palladium, Platin, Raney-Nickel. Als Reduktionsmittel verwendbare Hydride sind Natriumborhydrid und andere komplexe Hydride oder Diboran. Die Reaktionstemperatur liegt zwischen etwa 0 °C und der Siedetemperatur des Reaktionsgemischs. Sofern die Ausgangsstoffe eine hydrogenolytisch abspaltbare Schutzgruppe, z. B. eine gegebenenfalls substituierte Benzylgruppe aufweisen, wird diese während oder nötigenfalls nach der Reduktion der CO-Gruppe entfernt.

Die Ausgangsstoffe II gewinnt man beispielsweise durch Umsetzung von Bromketonen III mit Aminen IV in Lösungsmitteln wie Acetonitril oder Essigester in Gegenwart eines säureabfangenden Mittels wie Natriumcarbonat oder Aminüberschuß.

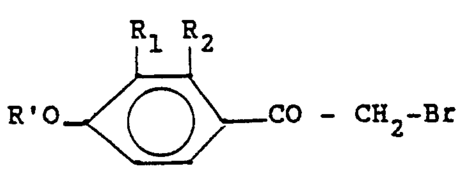

(III)

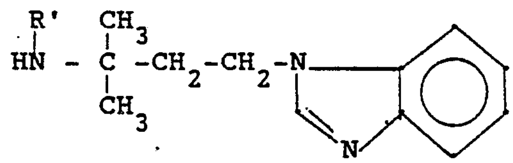

(IV)

R' bedeutet H oder einen hydrogenolytisch abspaltbaren Rest wie Benzyl, $R_1$ und $R_2$ sind wie oben definiert.

Verbindung VI kann in Verfahren b) auch in Form eines Halbacetals eingesetzt werden, d. h. in Form von Verbindungen der Formel

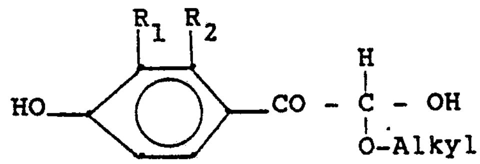

(VII)

worin $R_1$ und $R_2$ die oben angegebene Bedeutung haben und Alkyl für gegebenenfalls substituierte vorzugsweise $C_1$-$C_6$-Alkylreste steht.

Die eventuell als Zwischenprodukte auftretenden Schiffschen Basen VIII :

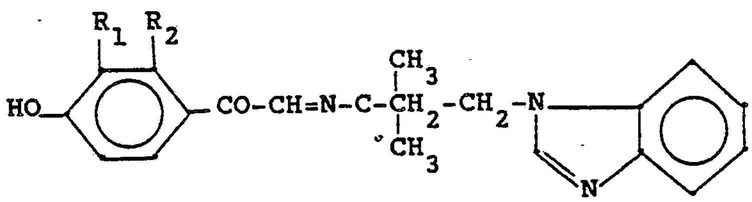

(VIII)

worin die einzelnen Symbole wie oben definiert sind, können auch isoliert und dann der Reduktion unterworfen werden.

Als Reduktionsmittel werden komplexe Hydride, vorzugsweise Natriumborhydrid, oder Wasserstoff in Gegenwart von Hydrierungskatalysatoren wie Platin, Palladium oder Raney-Nickel verwendet.

Etwa noch vorhandene Benzylschutzgruppen werden nötigenfalls nach der Reduktion auf übliche Weise entfernt.

Die Amine IV sind beispielsweise zugänglich, indem man Benzimidazol in an sich bekannter Weise mit einer Verbindung der Formel

$$B - C(CH_3)_2 - CH_2 - CH_2 - X \qquad (IX)$$

X : Chlor, Brom, Methyl- oder Tolylsulfonsäurerest ; B : funktionelle Gruppe wie $NO_2$, Benzalamino, die durch katalytische Hydrierung bzw. Hydrolyse in die Aminogruppe übergeführt wird, umsetzt.

Die als Ausgangsstoffe dienenden Phenylglyoxale VI bzw. die entsprechenden Halbacetale VII können beispielsweise durch Oxidation der Acetophenone X :

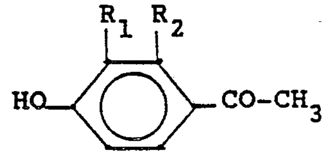

(X)

($R_1$ und $R_2$ wie oben definiert)

mit Selendioxid in wäßrigem Dioxan und Kristallisation aus Wasser oder Alkoholen erhalten werden.

Bei Verfahren c) erfolgt die Abspaltung der Schutzgruppen mit Wasserstoff und Hydrierungskatalysatoren wie Palladium, Platin, Raney-Nickel bei Temperaturen zwischen 0 °C und der Siedetemperatur des Reaktionsgemischs. Als Lösungsmittel dienen vorzugsweise niedere Alkohole, vor allem Methanol.

Die Ausgangsstoffe XI können entsprechend Verfahren a) oder b) hergestellt werden. Eine andere Möglichkeit besteht darin, in eine Verbindung XII :

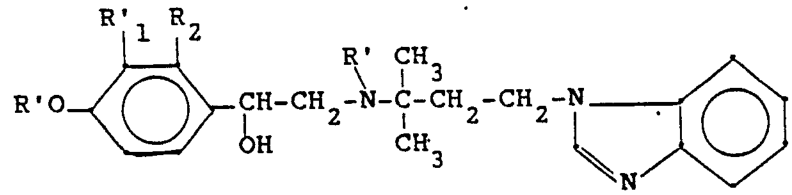

(XII)

worin R' Wasserstoff oder Benzyl, $R'_1$ für Vorstufen von $CH_2OH$, $NHSO_2CH_3$, $NHCOCH_3$, $NHCONH_2$ oder CN steht, die Gruppe $R'_1$ in $R_1$ umzuwandeln und/oder die Gruppe R' gleich Benzyl abzuspalten. Beispielsweise ergeben Verbindungen mit $R'_1$ gleich $NH_2$ mit Kaliumcyanat Verbindungen mit $R_1$ gleich $NHCONH_2$, mit $(CH_3CO)_2O$ wird $R_1$ gleich $NHCOCH_3$ erhalten. Verbindungen mit $R'_1$ gleich $COOC_2H_5$ ergeben durch Reduktion mit Lithiumaluminiumhydrid die entsprechenden Verbindungen mit $R_1$ gleich $CH_2OH$.

Die erfindungsgemäßen Verbindungen sind als Arzneistoffe verwendbar. Sie haben insbesondere broncholytische, spasmolytische und antiallergische Wirkung und können daher bei Bronchitis und Asthma, bei Urticaria, Conjunktivitis, Heufieber und Erkältungskrankheiten angewendet werden, ferner als Relaxantien der Uterusmuskulatur, z. B. bei Beschwerden vor der Geburt.

Hervorzuheben ist die starke und lange anhaltende broncholytische Wirkung, die mit nur geringer Nebenwirkung auf Herz und Skelettmuskulatur verbunden ist.

Die therapeutische Dosis ist abhängig von der verwendeten Verbindung, von der Art des Krankheitszustandes, von der Verabreichungsart und auch vom Körpergewicht, sofern nicht eine örtliche Anwendung erfolgt.

Für einen Erwachsenen kommen die folgenden Dosierungen pro Tag in Betracht :

Zur Broncholyse : oral 2-20 mg, inhalativ 0,1-1,5 mg, subcutan 0,2-1,5 mg. Zur Uterusspasmolyse : oral 10-50 mg, als Infusionslösung 0,1-1 mg in Ampullen mit 10 ml Lösung. Zur Gefäßerweiterung können oral 20-100 mg, in Form von Lösungen für die intramuskuläre Injektion 20-40 mg, zur Blutdrucksenkung oral 200-1 800 mg gegeben werden.

Für die Verabreichung werden aus den erfindungsgemäßen Verbindungen die üblichen galenischen Zubereitungen hergestellt, z. B. Kapseln, Tabletten, Dragées, Lösungen, Suspensionen, Pulver, Crems, Salben, Emulsionen und Sprays. Bei der pulmonalen Gabe werden vorzugsweise Pulver mit einem Teilchendurchmesser von 0,5 bis 7 µm als Aerosol mit der Atemluft, gegebenenfalls auch mit zusätzlichen Treibgasen, in den Bronchialbereich gebracht.

Die parenterale Anwendung erfolgt bevorzugt in Form steriler isotonischer wässeriger Lösungen, während für die lokale Anwendung vor allem Lotionen, Crems, Salben, Emulsionen und Sprays dienen.

Die günstigen Wirkungsverhältnisse bei den erfindungsgemäßen Verbindungen werden durch die nachstehenden Daten belegt.

1. Bronchospasmolyse

Die Wirkung wurde an Meerschweinchen in Urethan-Narkose geprüft. Bestimmt wurde durch Körperplethysmographie die Beeinflussung des Acetylcholin-Bronchospasmus nach intravenöser und oraler Applikation. Außerdem wurde die Herzfrequenz kontrolliert.

Die Verbindungen sind im folgenden mit den nachstehend aufgeführten römischen Zahlen bezeichnet, die der Gruppe

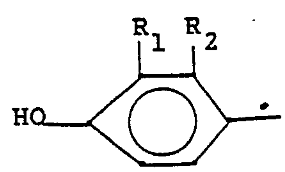

zugeordnet sind :

I

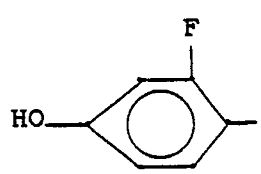

II

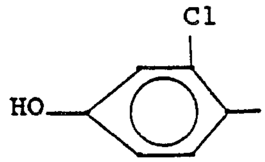

III

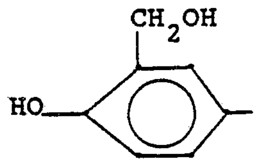

IV

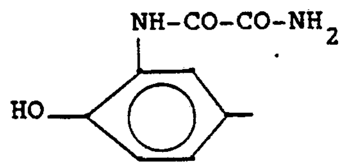

V

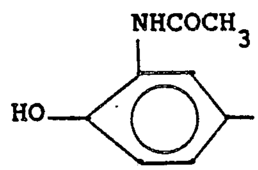

VI

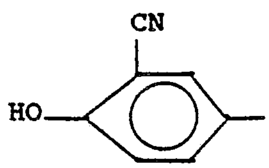

VII

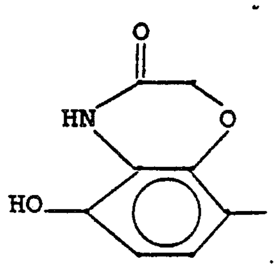

Broncholyse $ED_{50}$ µg/kg

| Verbindung | intravenös | oral | Resorptionsverhältnis oral / intravenös |
|---|---|---|---|
| I | 3,6 | 7 | 1,9 |
| II | 7,6 | 190 | 25 |
| III | 1,2 | 7,6 | 6,3 |
| IV | 0,9 | 14 | 16 |
| V | 1,8 | 66 | 37 |
| VII | 0,09 | 5,4 | 60 |
| Salbutamol | 9,2 | 1000 | 109 |

Die erfindungsgemäßen Verbindungen zeigen ein hervorragendes Verhältnis zwischen intravenöser und oraler Wirkung. Der Einfluß auf die Herzfrequenz ist gleichzeitig gering. Außerdem ist die Toxizität so niedrig, daß sich eine beträchtliche therapeutische Breite ergibt ; z. B. ist für die Verbindung I die $LD_{50}$ an der Maus i. v. 29 mg/kg, p. o. 330 mg/kg.

2. Uterusrelaxation

Die Uterusrelaxation wurde an narkotisierten Ratten untersucht. Bestimmt wurde die intravenöse $ED_{50}$ der Uterusrelaxation in µg/kg (50 % der geprüften Tiere reagieren) sowie die (unerwünschte) Zunahme der Herzfrequenz beim $ED_{50}$-Wert. Vergleichsverbindung ist Fenoterol.

| Verbindung | Uterus-relaxation $ED_{50}$ [µg/kg] i.v. | Zunahme der Herzfrequenz [Schläge/ min.] | Zunahme der Herzfrequenz im Vergleich zu Fenoterol |
|---|---|---|---|
| VI | 0,76 | 18 | 1/2 |
| Fenoterol | 0,45 | 36 | 1 |

Die Verbindung gemäß der Erfindung bewirkt somit bei Applikation der $ED_{50}$ Uterusrelaxation eine erheblich geringere Steigerung der Herzfrequenz als das Handelsprodukt Fenoterol.

## Formulierungsbeispiele

Tabletten
Zusammensetzung einer Tablette

| | |
|---|---|
| Wirkstoff gemäß der Erfindung | 20 mg |
| kolloidale Kieselsäure | 10 mg |
| Milchzucker | 118 mg |
| Kartoffelstärke | 60 mg |
| Polyvinylpyrrolidon | 6 mg |
| Na-Celluloseglykolat | 4 mg |
| Magnesiumstearat | 2 mg |
| | 220 mg |

Ampullen
Zusammensetzung der Lösung pro Ampulle

| | |
|---|---|
| Wirkstoff gemäß der Erfindung | 10 mg |
| Sorbit | 40 mg |
| destill. Wasser | ad 10 ml |

Suppositorien
Zusammensetzung pro Suppositorium

| | |
|---|---|
| Wirkstoff gemäß der Erfindung | 100 mg |
| Suppositorienmasse (Kakaobutter) | 1 600 mg |
| | 1 700 mg |

Inhalationspulver

Pro Hartgelatine-Steckkapsel werden 0.5 mg Wirkstoff gemäß der Erfindung und 19,5 mg Lactose mit einem Teilchendurchmesser zwischen 0.5 und 7 µm eingefüllt.

Die erfindungsgemäßen Wirkstoffe können auch mit bekannten Wirkstoffen kombiniert werden ; für

die broncholytische Anwendung z. B. mit Theophyllinen, Parasympatholytika (z. B. Ipratropiumbromid), Sekretolytika (z. B. Bromhexin), muskulotropen Spasmolytika (z. B. Papaverin), Corticosteroiden, Antiallergika. Bei den Uterusrelaxantien sind u. a. Kombinationen mit Corticoiden möglich.

Die erfindungsgemäßen Verfahren sind in den nachstehenden Beispielen näher erläutert. Die Ausbeuteangaben in den Tabellen stellen % der Theorie dar.

Verfahren a) kann analog den nachstehenden Mustern durchgeführt werden :

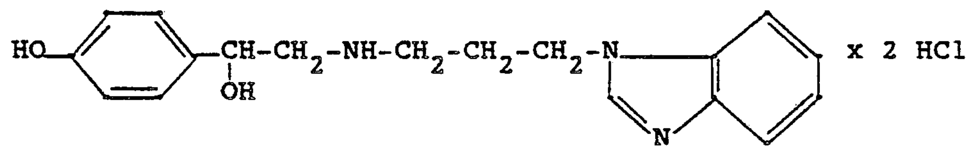

(1)

30,5 g 2-Brom-p-benzyloxacetophenon und 35 g 1-Aminopropylbenzimidazol werden in 150 ml Acetonitril 1 Stunde bei 30-40 °C gerührt. Nach dem Abtrennen des Hydrobromids wird die Mutterlauge mit 12 g Maleinsäure angesäuert und das ausgefallene α-[3-(1-Benzimidazolyl)-propyl-amino]-4-benzyloxyacetophenonmaleinat (Fp. 145-148 °C) abgesaugt. Mit wäßrigem Ammoniak wird daraus die Base hergestellt, die man in 200 ml Alkohol mit Natriumborhydrid zu 1-(4-Benzyloxyphenyl)-2-[3-(1-benzimidazolyl)-propylamino]-äthanol (Fp. 83-85 °C reduziert).

Durch katalytische Hydrierung von 7 g dieser Verbindung in 100 ml Methanol mit 1 g Palladiumkohle als Katalysator erhält man 4,5 g 1-(4-Hydroxyphenyl)-2-[3-(1-benzimidazolyl)-propylamino]-äthanol (Fp. 146-148 °C, Ausbeute 83 % d. Th.), dessen Dihydrochlorid bei 184-185 °C schmilzt.

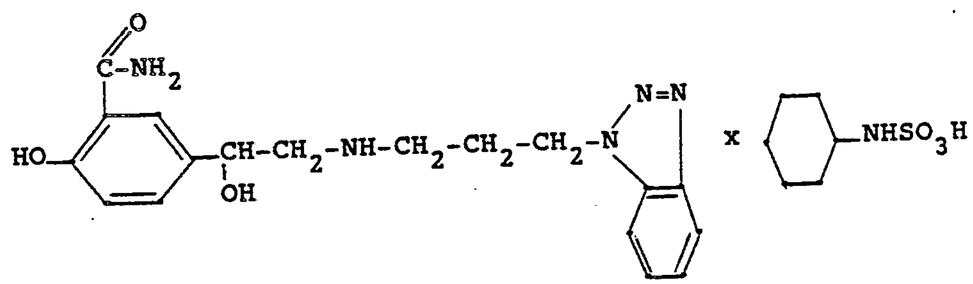

(2)

17,5 g 2-Benzyloxy-5-bromacetyl-salicylamid, 17,6 g 1-(3-Aminopropyl)-1H-benztriazol, 6 g Natriumcarbonat und 150 ml Essigester werden 1,5 Stunden refluxiert. Nach dem Abtrennen der anorganischen Bestandteile wird die Mutterlauge eingeengt, der Rückstand in 100 ml Acetonitril gelöst und mit 5 g Oxalsäure angesäuert. Das ausgefallene 1-[3-(3-Carbamoyl-4-benzyloxy-β-oxophenyläthylamino)-propyl]-1-H-benztriazol-oxalat wird abgesaugt, mit wäßrigem Ammoniak in die Base (Fp. 186-188 °C) überführt und in 100 ml Äthanol mit Natriumborhydrid zu 1-(3-Carbamoyl-4-benzyloxy-β-hydroxyphenäthylamino)-propyl-1-H-benztriazol reduziert.

Durch Hydrierung von 6 g dieser Verbindung in 100 ml Methanol bei 6 bar Druck und 40 °C unter Zusatz von Palladiumkohle erhält man 3 g 1-[3-(3-carbamoyl-4-β-dihydroxyphenäthylamino)-propyl]-1-H-benztriazol (Fp. 154-155 °C, Ausbeute 77,5 % d. Th.), dessen Cyclamat bei 165 °C schmilzt.

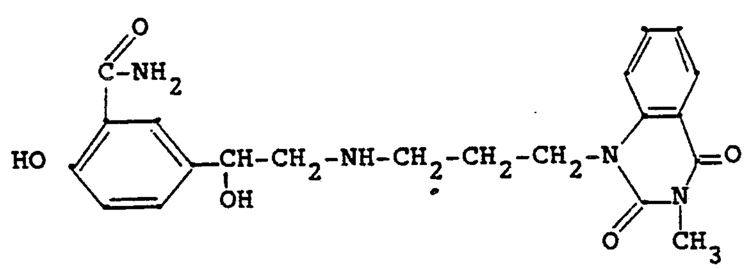

(3)

12,9 g 5-Bromacetylsalicylamid, 15,45 g 1-(3-Benzylaminopropyl)-3-methyl-chinazolin-2,4-dion, 6 g Natriumcarbonat und 300 ml Acetonitril werden 1,5 Stunden am Rückfluß gekocht. Nach dem Absugen der anorganischen Bestandteile wird die Mutterlauge eingeengt, der Rückstand in 500 ml Methanol gelöst und nach Zugabe von 12 ml Benzylchlorid bei 6 bar und 60 °C mit Palladiumkohle als Katalysator hydriert. Nach Aufnahme von 2 Mol Wasserstoff wird die Hydrierung aufgearbeitet und das entstandene 1-[3-(3-Carbamoyl-β-oxo-4-hydroxyphenäthylamino)-propyl]-3-methyl-chinazolin-2,4-dion-hydrochlorid (Fp. 253 °C Zers.) isoliert.

Durch katalytische Hydrierung von 13 g dieser Verbindung in 250 ml Methanol/Wassergemisch 1 : 1

bei 6 bar Druck, 50 °C mit Palladium als Katalysator erhält man 8 g 1-[3-(Carbamoyl-4-β-dihydroxyphenäthylamino)-propyl]-3-methyl-chinazolin-2,4-dion-hydrochlorid, dessen Schmelzpunkt 220-221 °C beträgt. Ausbeute : 61,5 % d. Th.

Erfindungsgemäß : Beispiel 1

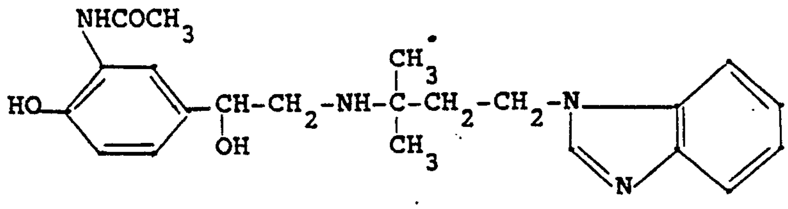

| Ausbeute (%) | Salz mit | Schmelzpunkt in °C |
|---|---|---|
| 60 | 1,5 x Bernsteinsäure | 154 - 156 |

Zu Verfahren b) : Beispiel 2

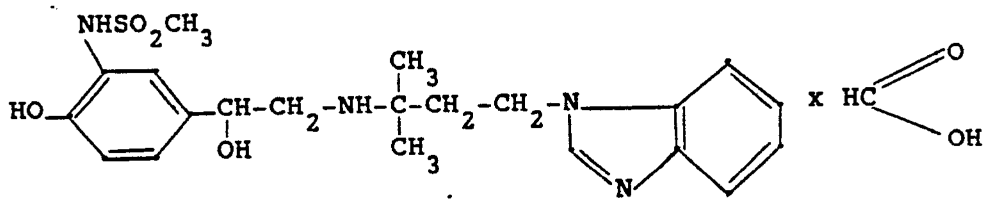

14,4 g 2'-Benzyloxy-5'-(1-oxo-2-hydroxy-2-äthoxy-äthyl)-metansulfonanilid, 7 g 1-(3-Amino-3-methylbutyl)-benzimidazol und 150 ml Alkohol werden 3 Stunden auf 50 °C erwärmt und portionsweise mit 9,2 g Natriumborhydrid versetzt. Die Lösung wird 12 Stunden bei Raumtemperatur gehalten, dann wird der Alkohol unter vermindertem Druck am Rotavapor entfernt und der Rückstand mit 200 ml Wasser und 500 ml Essigester gelöst. Nach dem Zersetzen des Natriumborhydrids unter Rühren mit konz. Salzsäure unter Eiskühlung wird mit wäßrigem Ammoniak alkalisch gestellt, die Essigester-Phase abgetrennt, mit Natriumsulfat getrocknet und am Rotavapor eingeengt. Der Rückstand wird in 200 ml Alkohol gelöst, mit 6,3 g Oxalsäure angesäuert und das ausgefallene 2'-Benzyloxy-5'-[1-hydroxy-2-[4-(1-benzimidazolyl)-2-methyl-2-butylamino]-äthyl]-methansulfonanilid-dioxalat (Fp. 185-187 °C) abgesaugt. Mit wäßrigem Ammoniak wird aus dieser Verbindung die Base (Fp. 65-70 °C) hergestellt. Durch katalytische Hydrierung dieser Verbindung (Fp. 65-70 °C) in 250 ml Methanol unter Normalbedingungen mit Palladiumkohle als Katalysator erhält man 8 g 2'-Hydroxy-5'-[1-hydroxy-2-[4-(1-benzimidazolyl)-2-methyl-2-butylamino]-äthyl]-methansulfonanilid (Fp. 170-173 °C, Ausbeute : 81 % d. Th.), dessen Formiat bei 161-164 °C schmilzt.

Beispiel 3

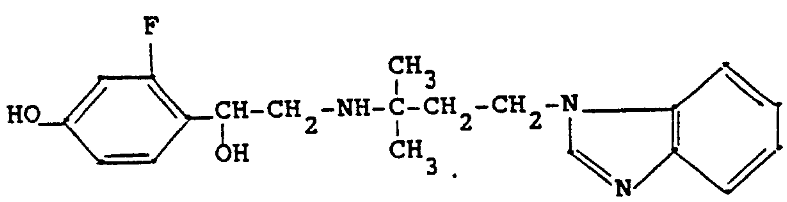

x HCOOH

15,2 g 1-(2-Fluor-4-benzyloxyphenyl)-1-oxo-2-hydroxy-2-äthoxy-äthan, gelöst in 200 ml Alkohol, werden mit 8,2 g 1-(3-Amino-3-methyl-butyl)-benzimidazol versetzt und 3 Stunden bei 50 °C gerührt. Anschließend wird die Lösung abgekühlt, mit 4 g Natriumborhydrid versetzt und 6 Stunden bei Raumtemperatur gerührt. Der Ansatz wird wie in Muster (3) beschrieben aufgearbeitet. Der Rückstand wird in 200 ml Alkohol gelöst mit 7,2 g Oxalsäure angesäuert und das ausgefallene 1-(2-Fluor-4-benzyloxyphenyl)-2-[4-(1-benzimidazolyl)-2-methyl-2-butylamino]-äthanol-dioxalat (Fp. 184-188 °C abgesaugt). Mit wäßrigem Ammoniak wird aus dem Dioxalat die Base (Fp. 85-88 °C) hergestellt.

Durch katalytische Hydrierung dieser Verbindung in 250 ml Methanol bei 6 bar Druck mit Palladiumkohle als Katalysator bei ~ 30 °C erhält man 1-(2-Fluor-4-hydroxyphenyl)-2-[4-(1-benzimidazolyl)-2-methyl-2-butylamino]-äthanol (Fp. 151-153 °C), dessen Formiat bei 157-159 °C schmilzt.

Durch Zugabe der berechneten Menge Methansulfonsäure zu der Lösung der Base in Äthanol erhält man das Methansulfonat, Fp. 178-179 °C. Entsprechend stellt man das Hydrochlorid her, Fp. 205 °C.

Aus der Base in wäßrigem Acetonitril und der berechneten Menge konz. Salzsäure erhält man das Dihydrochlorid-Tetrahydrat, Fp. 177 °C.

Analog gelant man in wäßrigem Äthanol zum Sulfat-Hydrat, Fp. 207-208 °C.

(Seihe Tabelle Siete 11 f.)

| Formel | Ausbeute (%) | Salz mit | Schmelzpunkt in °C |
|---|---|---|---|
| 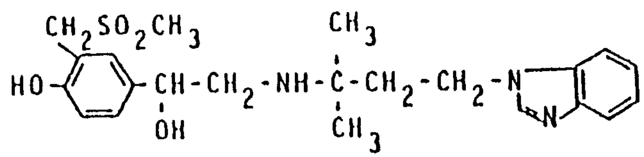 | 93 | Maleinsäure | 190 – 193<br>181 – 183 Base |
| 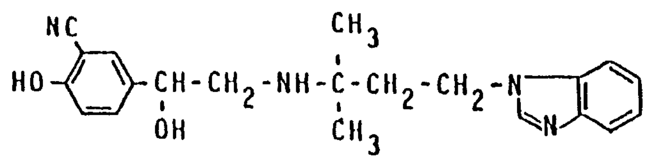 | 48,5 | 2 × Salzsäure | 208 – 210 Zers. |
| 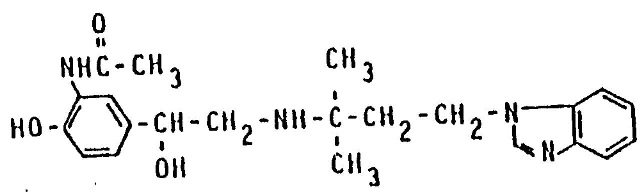 | 50 | 1,5 × Bernsteinsäure | 154 – 156 |
| 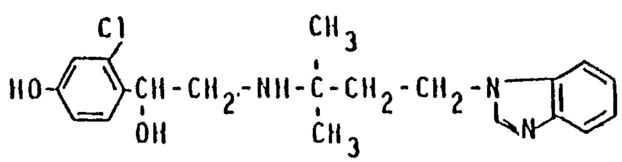 | 80 | Maleinsäure | 185 – 186<br>175 – 178 Base |

Muster für Verfahren c)

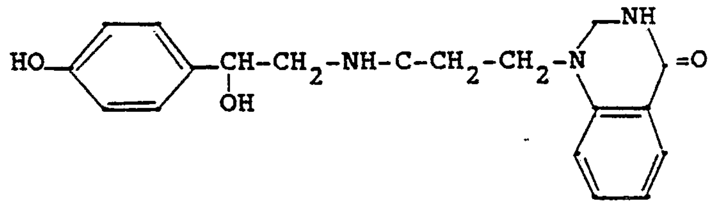

x HCOOC (1)

15,5 g 1-{-[2-Hydroxy-2-(4-benzyloxyphenyl)-benzyl-äthylamino]-propyl}-1,2,3,4,-tetrahydro-4-chinazolon (Fp. 119-121 °C) werden in 250 ml Methanol gelöst und bei 60 °C, 6 bar Druck unter Zusatz von Palladium als Katalysator entbenzyliert. Nach Aufnahme von 2 Mol Wasserstoff wird die Hydrierung aufgearbeitet, wobei man 8 g 1-{3-[2-Hydroxy-2-(4-hydroxyphenyl)-äthylamino]-propyl}-1,2,3,4-tetrahydro-4-chinazolon erhält, dessen Formiat bei 174-176 °C schmilzt. Die Ausbeute beträgt 61,5 % d. Th.

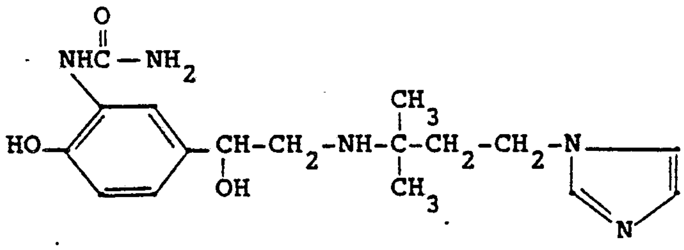

x $H_2SO_4$ (2)

15 g 2'-Benzyloxy-5'-[1-hydroxy-2-[4-(1-imidazolyl)-2-methyl-2-butylamino]-äthyl]-acetanilid (Fp. des Dioxalats 160-163 °C), 18,5 g KOH, 80 ml Alkohol und 15 ml Wasser werden 24 Stunden unter Rückfluß gekocht und anschließend das entstandene 1-(3-Amino-4-benzyloxy-phenyl-2-[4-(1-imidazolyl)-2-methyl-2-butylamino]-äthanol als Trioxalat (Fp. 95-100 °C) isoliert. Mit wäßrigem Ammoniak wird daraus die Base hergestellt, die man mit Kaliumcyanat zu 1-{2'-Benzyloxy-5'-[1-hydroxy-2-[4-(1-imidazolyl)-2-methyl-2-butylamino]-äthyl]-phenyl}-harnstoff (Fp. 142-143 °C) umsetzt. Durch katalytische Hydrierung von 5,1 g dieser Verbindung in 100 ml Methanol mit Palladiumkohle als Katalysator erhält man 3,5 g 1-{2'-Hydroxy-5'-[1-hydroxy-2-]4-(1-imidazolyl)-2-methyl-2-butylamino]-äthyl]-phenyl}-harnstoff, dessen Sulfat bei 243-244 °C schmilzt. Die Ausbeute beträgt 70 % d. Th.

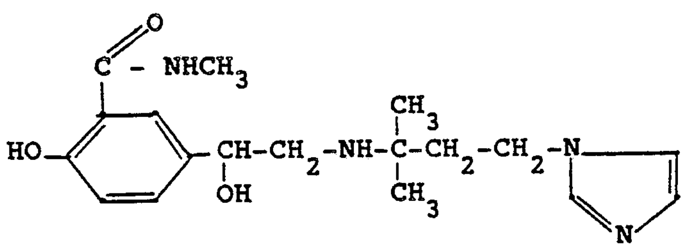

x $H_2SO_4$ (3)

8,5 g 2'-Benzyloxy-5'-[1-hydroxy-2-[4-(1-imidazolyl)-2-methyl-4-butylamino]-äthyl]-benzoesäuremethylester (Fp. des Dioxalats 156-158 °C) werden unter Zusatz von Palladium als Katalysator in 100 ml Methanol unter Normalbedingungen hydriert, nach Aufnahme von 1 Mol Wasserstoff wird der Katalysator abfiltriert und zu der Lösung 15 ml Monomethylamin zugefügt. Nach 2 Tagen wird das Lösungsmittel abdestilliert, der Rückstand mit 15 ml Alkohol und 15 ml Wasser gelöst und mit 2 g konz. Schwefelsäure angesäuert. Es werden 4,5 g 5-[1-Hydroxy-2-[4-(1-imidazolyl)-2-methyl-2-butylamino]-äthyl]-N-methyl-salicylamid-Sulfat mit dem Schmelzpunkt von 263-265 °C (Zers.) isoliert. Die Ausbeute beträgt 52 % d. Th.

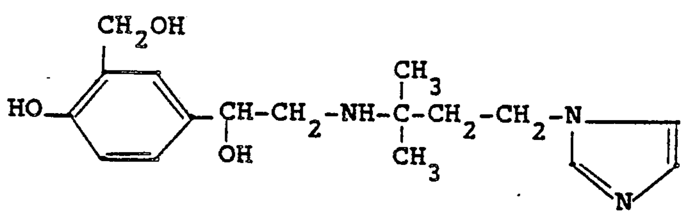

x $C_6H_5COOH$ (4)

13,5 g 2'-Benzyloxy-5'-[1-hydroxy-2-[4-(1-imidazolyl)-2-methyl-4-butylamino]-äthyl]-benzoesäuremethylester (Fp. des Dioxalats 156-158 °C) werden in 200 ml Tetrahydrofuran mit 6 g Lithiumaluminiumhydrid zu 1-(3-Hydroxymethyl-4-benzyloxyphenyl)-2-[4-(1-imidazolyl)-2-methyl-2-butylamino]-äthanol reduziert, dessen Dioxalat bei 144-146 °C schmilzt. Mit wäßrigem Ammoniak wird aus 10 g Dioxalat die Base hergestellt und diese mit Palladium als Katalysator in 100 ml Methanol hydriert. Man erhält 4,5 g 1-(3 Hydroxymethyl-4-hydroxyphenyl)-2-[4-(1-imidazolyl)-2-methyl-2-butyl-amino]-äthanol (Fp. 135-137 °C), dessen Benzoat bei 150-152 °C schmilzt. Die Ausbeute beträgt 83 % d. Th.

In analoger Weise hergestellt :

| Formel | Ausbeute (%) | Salz mit | Schmelzpunkt in ° C |
|---|---|---|---|
| 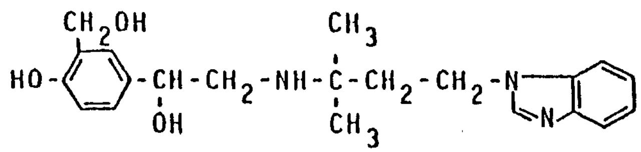 | 52 | . Cyclohexan-sulfaminsäure | 126 – 130 |
| 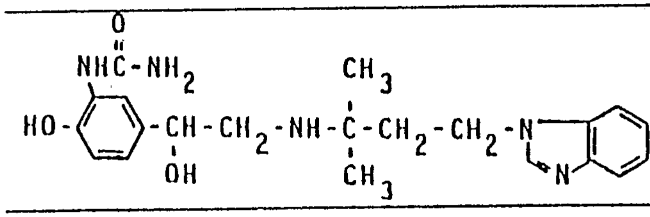 | 45 | Maleinsäure | 186 – 188 |
| 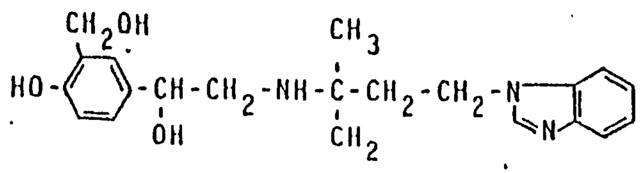 | 52 | Cyclohexan-sulfaminsäure × 1 Wasser | 126 – 130 |

**Patentansprüche**

1. Verbindungen der allgemeinen Formel

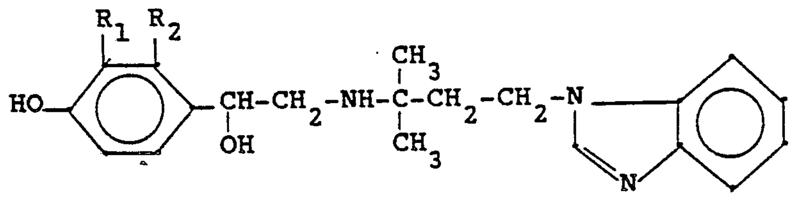

(I)

in der
$R_1$ $CH_2OH$, $NHSO_2CH_3$, $NHCOCH_3$, $NHCONH_2$ und CN bedeutet und $R_2$ für H steht oder
$R_1$ H bedeutet und $R_2$ für F oder Cl steht oder
$R_1$ und $R_2$ gemeinsam für $—O—CH_2—CO—NH—$ in 2,3-Position stehen, in Form der Racemate oder Enantiomeren und der jeweiligen Säureadditionssalze.

2. Verbindungen nach Anspruch 1, dadurch gekennzeichnet, daß $R_1/R_2$ die Substituentenkombinationen $CH_2OH/H$, $NHCONH_2/H$, H/F oder H/Cl bedeuten, in Form der Racemate oder Enantiomeren und der jeweiligen Säureadditionssalze.

3. Verbindung nach Anspruch 1, dadurch gekennzeichnet, daß $R_1$ Wasserstoff, $R_2$ Fluor bedeutet, in Form des Racemats, der Enantiomeren und der jeweiligen Säureadditionssalze.

4. Arzneimittel gekennzeichnet durch einen Gehalt an einer Verbindung nach Anspruch 1, 2 oder 3.

5. Verfahren zur Herstellung von Verbindungen der allgemeinen Formel

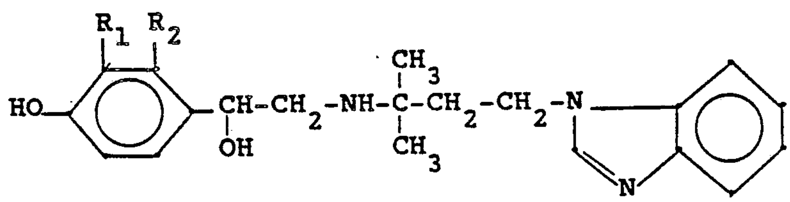

(I)

in der
$R_1$ $CH_2OH$, $NHSO_2CH_3$, $NHCOCH_3$, $NHCONH_2$ und CN bedeutet und $R_2$ für H steht oder
$R_1$ H bedeutet und $R_2$ für F oder Cl steht oder
$R_1$ und $R_2$ gemeinsam für $—O—CH_2—CO—NH—$ in 2,3-Position stehen, in Form der Racemate oder Enantiomeren und der jeweiligen Säureadditionssalze in an sich bekannter Weise,
dadurch gekennzeichnet, daß man

a) ein Aminoketon der allgemeinen Formel.

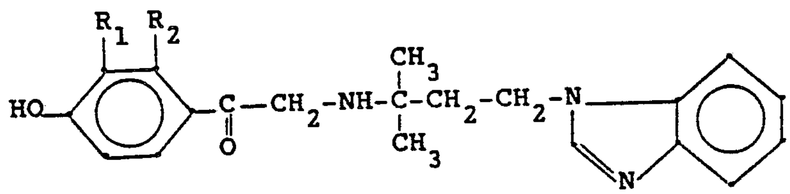

(II)

in der $R_1$ und $R_2$ die oben angegebene Bedeutung haben und in der das zentrale Stickstoffatom und/oder die phenolische OH-Gruppe durch hydrogenolytisch abspaltbare Schutzgruppen geschützt sein können, in einem geeigneten Lösungsmittel mit Wasserstoff und Hydrierungskatalysatoren oder mit reduzierend wirkenden Hydriden reduziert und gegebenenfalls nach der Reduktion noch vorhandene Schutzgruppen hydrogenolytisch entfernt oder daß man

b) das Amin der Formel

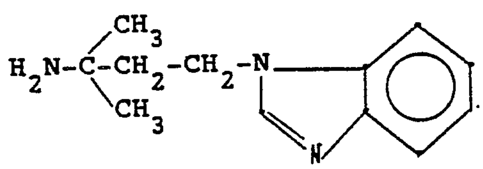

(V)

unter den Bedingungen der reduktiven Aminierung mit einem Phenylglyoxal der allgemeinen Formel

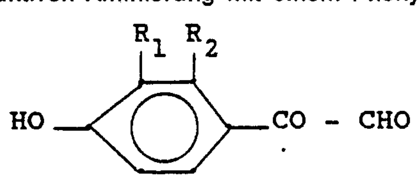

(VI)

in der $R_1$ und $R_2$ die obige Bedeutung haben und in der die phenolische OH-Gruppe durch eine hydrogenolytisch abspaltbare Schutzgruppe geschützt sein kann, gegebenenfalls in Form eines Halbacetals umsetzt und gegebenenfalls die Schutzgruppe hydrogenolytisch abspaltet oder daß man

c) aus einer Verbindung der allgemeinen Formel

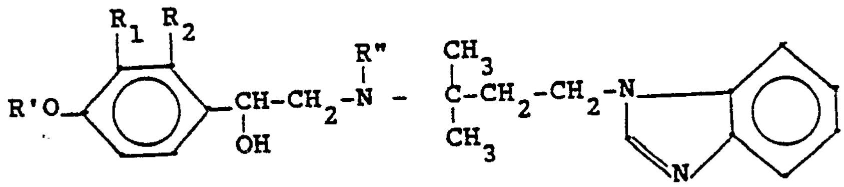

(XI)

in der $R_1$ und $R_2$ die obige Bedeutung haben, R′ und R″ für H oder eine hydrogenolytisch abspaltbare Schutzgruppe stehen, wobei mindestens einer der Reste R′ und R″ eine Schutzgruppe bedeutet, die Schutzgruppe (n) entfernt

und daß man die nach a) bis c) erhaltenen Verbindungen gewünschtenfalls in die Enantiomeren auftrennt und daß man gewünschtenfalls erhaltene Basen in Säureadditionssalze, erhaltene Säureadditionssalze in die freien Basen oder in Salze mit anderen Säuren überführt.

6. Verbindungen gemäß Anspruch 1 zur Verwendung bei der Behandlung von Bronchospasmen oder Uterusspasmen.

## Claims

1. Compounds of general formula

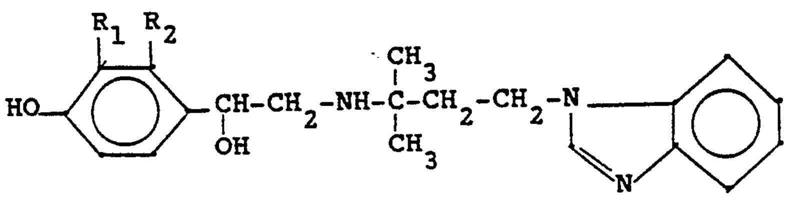

(I)

wherein

$R_1$ represents $CH_2OH$, $NHSO_2CH_3$, $NHCOCH_3$, $NHCONH_2$ and $R_2$ represents H or

$R_1$ represents H and $R_2$ represents F or Cl or

$R_1$ and $R_2$ together represent $—O—CH_2—CO—NH—$ in 2,3-position) in the form of racemates or enantiomers and the acid addition salts thereof.

2. Compounds as claimed in claim 1 characterized in that $R_1/R_2$ represent the combinations of substituents $CH_2OH/H$, $NHCONH_2/H$, H/F or H/Cl, in the form of racemates or enantiomers and the acid addition salts thereof.

3. Compounds as claimed in claim 1, characterized in that $R_1$ represents hydrogen, $R_2$ represents fluor, in the form of the racemate or enantiomer and the acid addition salts thereof.

4. Pharmaceutical compositions, characterized in that they contain a compound as claimed in claim 1, 2 or 3.

5. Process for the preparation of compounds of general formula

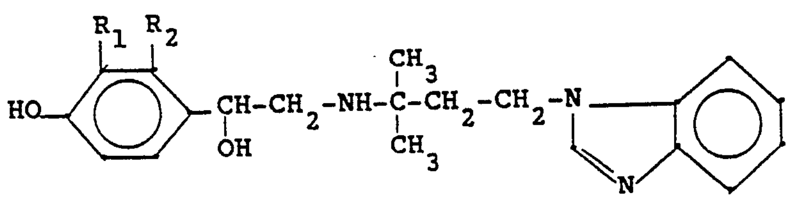

(I)

wherein

$R_1$ represents $CH_2OH$, $NHSO_2CH_3$, $NHCOCH_3$, $NHCONH_2$ and $R_2$ represents H or

$R_1$ represents H and $R_2$ represents F or Cl or

$R_1$ and $R_2$ together represent $—O—CH_2—CO—NH—$ in 2, 3-position) in the form of racemates or enantiomers and the acid addition salts thereof

in a manner known per se, characterized in that

a) an aminoketone of general formula

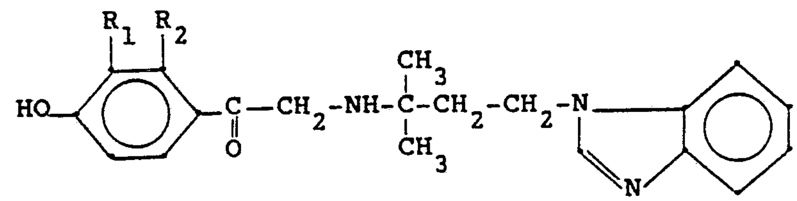

(II)

wherein $R_1$ and $R_2$ are as hereinbefore defined and wherein the central nitrogen atom and/or any phenolic OH groups present may be protected by hydrogenolytically cleavable protecting groups, is reduced in a suitable solvent with hydrogen and hydrogenation catalysts or with hydrides with a reducing effect, and if desired, after the reduction, any protecting groups still present are removed hydrogenolytically, or

b) the amine of formula

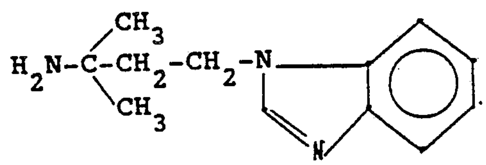

(V)

is reacted under the conditions of reductive amination, with a phenylglyoxal of general formula wherein $R_1$ and $R_2$ are as hereinbefore defined and wherein any phenolic OH groups present may be protected by hydrogenolytically cleavable protecting groups, the said phenylglyoxal optionally beeing reacted in the form of a hemiacetal, and any protecting groups present are cleaved hydrogenolytically, or

c) the protecting group or groups is removed from a compound of general formula

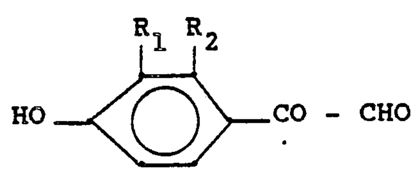

(VI)

wherein $R_1$ and $R_2$ are as hereinbefore defined, R′ and R″ represent H or a hydrogenolytically cleavable protecting group, and wherein at least one of the groups R′ and R″ represents a group which is to be removed,

and if desired the compounds obtained according to a) to c) may be resolved into the enentiomers, and if desired any bases obtained are converted into acid addition salts, or any acid addition salts obtained are converted into free bases or into salts with other acids.

6. Compounds according to claim 1, for use in the treatment of bronchospasms or uterine spasms.

**Revendications**

1. Composés de formule générale

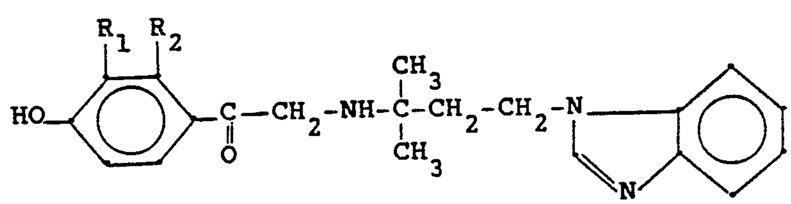

(I)

dans laquelle

$R_1$ signifie $CH_2OH$, $NHSO_2CH_3$, $NHCOCH_3$, $NHCONH_2$ et CN et $R_2$ remplace H ou

$R_1$ signifie H et $R_2$ remplace F ou Cl ou

$R_1$ et $R_2$ ensemble remplacent $-O-CH_2-CO-NH-$ en position 2, 3, sous forme des racémates ou des énantiomères et des sels d'addition d'acides correspondants.

2. Composés selon la revendication 1, caractérisés en ce que $R_1/R_2$ représentent les combinaisons de substituants $CH_2OH/H$, $NHCONH_2/H$, H/F ou H/Cl, sous forme des racémates ou des énantiomères et des sels d'addition d'acides correspondants.

3. Composés selon la revendication 1, caractérisés en ce que $R_1$ représente l'hydrogène, $R_2$ représente le fluor, sous forme du racémate, des énantiomères et des sels d'addition d'acides correspondants.

4. Médicament caractérisé par une teneur en un composé selon la revendication 1, 2 ou 3.

5. Procédé pour la préparation de composés de formule générale

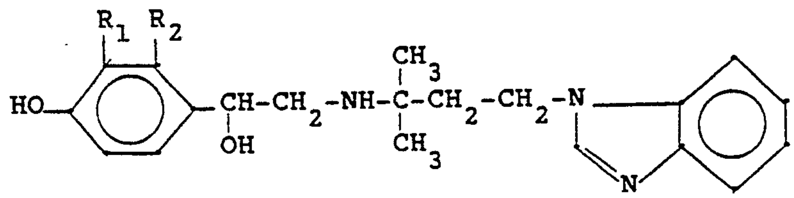

(I)

dans laquelle

$R_1$ signifie $CH_2OH$, $NHSO_2CH_3$, $NHCOCH_3$, $NHCONH_2$ et CN et $R_2$ remplace H ou

$R_1$ signifie H et $R_2$ remplace F ou Cl ou

$R_1$ et $R_2$ ensemble remplacent —O—$CH_2$—CO—NH— en position 2, 3, sous forme des racémates ou des énantiomères et des sels d'addition d'acides correspondants,

de manière en soi connue, caractérisé en ce que

a) on réduit une aminocétone de formule générale

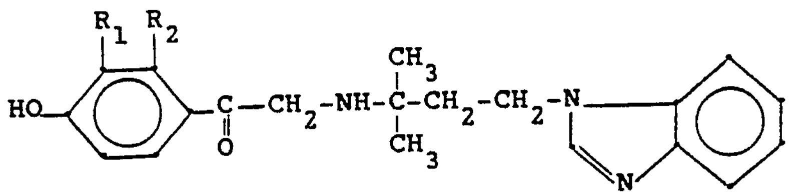

(II)

dans laquelle, $R_1$ et $R_2$ ont la signification donnée plus haut et dans laquelle l'atome d'azote central et/ou le groupe OH phénolique peuvent être protégés par des groupes protecteurs clivables hydrogénolytiquement, dans un solvant approprié au moyen d'hydrogène et de catalyseurs d'hydrogénation ou au moyen d'hydrures à activité réductrice et éventuellement, après la réduction, on élimine hydrogénolytiquement les groupes protecteurs encore présents ou en ce que

b) on fait réagir l'amine de formule

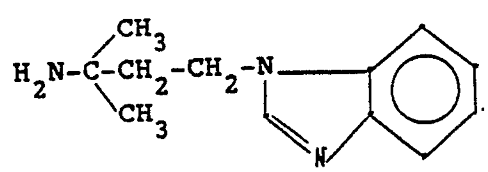

(V)

dans les conditions de l'amination réductrice, avec un phénylglyoxal de formule générale

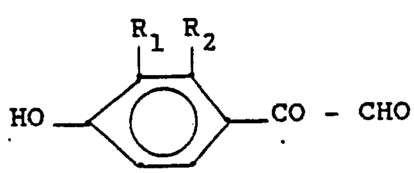

(VI)

dans laquelle, $R_1$ et $R_2$ ont la signification ci-dessus et dans laquelle le groupe OH phénolique peut être protégé par un groupe protecteur clivable hydrogénolytiquement, éventuellement sous forme d'un semiacétal, et éventuellement on clive hydrogénolytiquement le groupe protecteur ou en ce que

c) on élimine le ou les groupe(s) protecteur(s) à partir d'un composé de formule générale

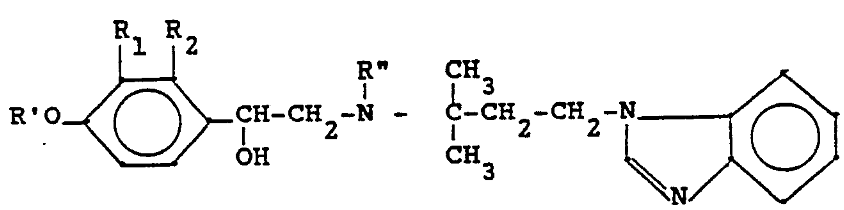

(XI)

dans laquelle, $R_1$ et $R_2$ ont la signification ci-dessus, R' et R" remplacent H ou un groupe protecteur clivable hydrogénolytiquement, au moins l'un des radicaux R' et R" représentant un groupe protecteur, et en ce qu'on sépare si on le désire les composés obtenus selon a) à c) en les énantiomères et en ce que, si on le désire, on transforme les bases obtenues en sels d'addition avec des acides, les sels d'addition avec des acides obtenus en les bases libres ou en sels avec d'autres acides.

6. Composés selon la revendication 1 pour l'utilisation dans le traitement des bronchospasmes ou des spasmes utérins.